# EUROPEAN PATENT SPECIFICATION

(11) **EP 3 320 848 B1**
(45) Date of publication and mention of the grant of the patent: **27.03.2019**
(21) Application number: 16198311.9
(22) Date of filing: 11.11.2016
(51) Int. Cl.: A61B 8/00, A61B 90/14, A61B 8/08

(54) **POSITIONING APPARATUS FOR HEAD AND NECK ASSESSMENT OR INTERVENTION**
POSITIONIERUNGSVORRICHTUNG FÜR KOPF- UND NACKENBEURTEILUNG ODER -EINGRIFF
APPAREIL DE POSITIONNEMENT POUR ÉVALUATION OU INTERVENTION SUR LA TÊTE ET LE COU

(43) Date of publication of application: 16.05.2018
(73) Proprietor: AmCad BioMed Corporation, 105 Taipei City (TW)
(72) Inventor: Chen, Argon, 105 Taipei City (TW); Wang, Hao-Chien, 100 Taipei City (TW); Chen, Chiung-Nien, 110 Taipei City (TW); Lee, Pei-Lin, 104 Taipei City (TW); Shu, Chin-Chung, 100 Taipei City (TW); Liu, Edward Chia-Hao, 105 Taipei City (TW); YU, Shu-Ning, 105 Taipei City (TW)
(74) Representative: Becker Kurig Straus

(56) References cited:
- WO-A1-98/17177
- CN-U- 205 094 475
- JP-A- 2008 036 046
- US-A1- 2015 297 176

## Description

### FIELD OF THE INVENTION

The present invention relates to a positioning apparatus for head and neck assessment or intervention, and in particular, to a positioning apparatus that can fix a patient's head in a position with reference to more than one desired reference planes of the patient.

### BACKGROUND OF THE INVENTION

US Patent No. 6,425,865 discloses a system for medical ultrasound in which the ultrasound probe is positioned by a robot arm under the shared control of the ultrasound operator and the computer.

US 2015/0297176 A1 discloses a head frame configured for a head of a medical patient comprising support for a probe configured for imaging, therapy, or both imaging and therapy; and a neck support of rigid construction, said neck support being configured for contacting, providing rigid support for, a neck of said patient.

JP 2008 036046 A discloses a positioning apparatus for head and neck assessment according to the preamble of claim 1.

CN 205 094 475 U discloses a head positioning apparatus comprising an ear aligning member and a first light transmitter for defining a reference plane. Yet, the transmitter is not connected to one of the lateral ends of a main body and a reference plane does not pass through the respective point of pivotal connection.

### SUMMARY OF THE INVENTION

The present invention provides a positioning apparatus for head and neck assessment or intervention of a subject, comprising: a base; a head fixation assembly including (i) a main body attached to the base, the main body having two upwardly extending lateral ends, (ii) a guide representing a first reference plane of the subject, the guide extending upward and slightly inward from each of the lateral ends, and (iii) an ear aligning member for aligning with the opening of ear canal of the subject, configured on an inner side of each of the lateral ends; a first connector having a first end and an opposite second end, the first end being pivotally connected to one of the lateral ends of the main body on an outer side at a first pivot point; a first light transmitter for projecting a first light beam, configured at the second end of the first connector, such that the first light beam defines a second reference plane passing through the first pivot point; and a holder assembly including an adjustable first stand secured to the base, and a holder for holding a medical device for the assessment or intervention, pivotally connected to the first stand.

In certain embodiments of the present invention, the positioning apparatus further comprises a second stand secured to the base and extending upward to an upper end; a second connector having a first end and an opposite second end, the first end being pivotally connected to the upper end of the second stand at a second pivot point; and a second light transmitter for projecting a second light beam, configured at the second end of the second connector, such that the second light beam defines a third reference plane passing through the second pivot point.

In certain embodiments of the present invention, the guide includes a line representing the first reference plane. In some embodiments, the guide includes a transparent substrate extending upward and slightly inward from each of the lateral ends. According to one specific embodiment of the present invention, the transparent substrate is marked with a line representing the first reference plane. In another embodiment, the transparent substrate is projected with a light beam which serves as a line representing the first reference plane.

The ear aligning member may have a protruding part for fitting into the ear canal of the subject. Preferably, the first pivot point lies on a longitudinal axis of the protruding part of the ear aligning member. The ear aligning member may include a third light transmitter which projects a linear light beam (e.g., a laser transmitter which projects a linear laser beam) for aligning with the ear canal of the subject. Preferably, the first pivot point lies on an imaginary line extended from said linear light beam.

Preferably, the head fixation assembly includes two ear aligning members. On the other hand, the head fixation assembly may be configured with one first light transmitter, or configured with two first light transmitters symmetrically on the two lateral ends of the main body.

The first reference plane can be a Frankfort horizontal plane of the subject.

Preferably, the first light transmitter is a laser transmitter which projects a linear laser beam, and the second light transmitter is also a laser transmitter which projects a linear laser beam.

According to certain embodiments of the present invention, the main body of the head fixation assembly is slidably attached to the base.

According to certain embodiments of the present invention, the head fixation assembly further includes a pillow member for supporting the head of the subject, the pillow member being disposed on the main body between its lateral ends. Preferably, the pillow member is adapted for adjusting a tilt angle of the head of the subject with respect to a transverse plane of the subject.

The second stand can be secured to the base at a securing point where a medial line of the head fixation assembly passes through. In such case, the third reference plane may be a medial plane of the subject.

According to certain preferred embodiments, the first stand includes a first arm extending upward from the base to an upper end; a second arm connected to the upper end of the first arm at a right angle and extending along a direction parallel to the medial line of the head fixation assembly to an inferior end; a third arm connected to the inferior end of the second arm at a right angle and extending along a direction perpendicular to the medial line of the head fixation assembly to an medial end; and a fourth arm connected to the medial end of the third arm at a right angle and extending downward to a lower end. Preferably, the length of each arm is adjustable. In one embodiment, the holder is pivotally connected to the lower end of the fourth arm. In some embodiments, the head fixation assembly further includes a pillow member for supporting the head of the subject, disposed on the main body between the lateral ends, the pillow member being adapted for adjusting a tilt angle of the head of the subject with respect to a transverse plane of the subject.

The positioning apparatus of the present invention may further comprise a computer module adapted to detect and memorize the length of said each arm and/or the position of the pillow member corresponding to a tilt angle of the head of the subject with respect to a transverse plane of the subject.

In some embodiments of the present invention, the holder assembly further includes a motor for rotating the holder with respect to the first stand.

In certain preferred embodiments of the present invention, the holder assembly further includes a cushioning element for cushioning the movement of the medical device relative to the holder.

According to certain embodiments of the present invention, the holder comprises a frame part and a holding part, the frame part being pivotally connected to the first stand, the holding part being pivotally connected to the frame part and adapted for holding the medical device. In one embodiment, the holder further comprises a motor for rotating the holding part with respect to the frame part. In another embodiment, the holding part includes a cushioning element for cushioning the movement of the medical device relative to the holding part.

According to one preferred embodiment of the present invention, the positioning apparatus is for use in the assessment of obstructive sleep apnea of the subject by ultrasound imaging. In such embodiment, the second reference plane may be a hyoid-external auditory meatus plane of the subject.

The positioning apparatus may also be used in at least the following assessments or interventions: ultrasound-guided interventions (e.g., puncture, injection, biopsy, and nerve block), and ultrasound imaging of peripheral arteriovenous of neck, carotid artery, cervical lymph node, thyroid, parathyroid or parotid.

These and other aspects will become apparent from the following description of the preferred embodiment taken in conjunction with the following drawings, although variations and modifications therein may be affected without departing from the spirit and scope of the novel concepts of the disclosure.

### BRIEF DESCRIPTION OF THE DRAWINGS

The foregoing summary, as well as the following detailed description of the invention, will be better understood when read in conjunction with the appended drawing. In the drawings:
Fig. 1 is a perspective view of a positioning apparatus according to the present invention.
Fig. 2 is a schematic drawing illustrating the first, second and third reference planes.
Fig. 3 is a perspective view of a holder of a positioning apparatus according to the present invention.
Fig. 4 illustrates the use of a positioning apparatus according to the present invention in the assessment of obstructive sleep apnea of a subject by ultrasound imaging.

### DESCRIPTION OF THE INVENTION

Unless defined otherwise, all technical and scientific terms used herein have the same meaning as commonly understood by a person skilled in the art to which this invention belongs.

As used herein, the singular forms "a", "an", and "the" include plural referents unless the context clearly dictates otherwise. Thus, for example, reference to "a sample" includes a plurality of such samples and equivalents thereof known to those skilled in the art.

The present invention provides a positioning apparatus for head and neck assessment or intervention of a subject. The positioning apparatus comprises a base, a head fixation assembly, a first connector, a first light transmitter, and a holder assembly. The head fixation assembly includes a main body, a guide representing a first reference plane of the subject, and an ear aligning member. The main body is attached to the base and has two upwardly extending lateral ends. The guide extends upward and slightly inward from each of the lateral ends. The ear aligning member is to be aligned with the opening of ear canal of the subject. The ear aligning member is configured on an inner side of each of the lateral ends. The first connector has a first end and an opposite second end. The first end is pivotally connected to one of the lateral ends of the main body on an outer side at a first pivot point. Accordingly, the first connector is rotatable with respect to the first pivot point. The first light transmitter is able to project a first light beam, and is configured at the second end of the first connector, such that the first light beam defines a second reference plane passing through the first pivot point. The holder assembly includes an adjustable first stand, and a holder for holding a medical device for the assessment or intervention. The first stand is secured to the base, and the holder is pivotally connected to the first stand.

The positioning apparatus of the present invention is generally designed for head and neck assessment or intervention of a subject in a lying down or leaning backward position. Therefore, the base of the positioning apparatus is preferably a plate-like base which is adapted to flat against and/or be fixed on a bed. The base can be configured with one or more belts on a lateral side thereof, which may be used to fix the base to the bed.

The ear aligning member may have a protruding part for fitting into the ear canal of the subject. Preferably, the first pivot point lies on a longitudinal axis of the protruding part of the ear aligning member. The ear aligning member may include a third light transmitter which projects a linear light beam (e.g., a laser transmitter which projects a linear laser beam) for aligning with the ear canal of the subject. Preferably, the first pivot point lies on an imaginary line extended from said linear light beam.

According to certain embodiments of the present invention, the main body of the head fixation assembly is slidably attached to the base.

In certain embodiments of the present invention, the guide includes a line representing the first reference plane. In some embodiments, the guide includes a transparent substrate extending upward and slightly inward from each of the lateral ends. According to one specific embodiment of the present invention, the transparent substrate is marked with a line representing the first reference plane. In another embodiment, the transparent substrate is projected with a light beam which serves as a line representing the first reference plane.

The guide is preferably retractable in length. For example, the transparent substrate may be strip-like and be adapted to slide into and out of the main body, such that the guide may be in part pushed into the lateral end of the main body for the convenience of placement of the subject's head on the main body between its lateral ends, and may be later drawn out for aligning an end point of the line with certain reference point on the subject's head when an operator is to adjust the position of the subject's head with reference to the line of the guide.

The ear aligning member and the first connector can be coaxially connected to the main body, such that the first pivot point, where the first connector is pivoted to the lateral end of the main body, lies on the longitudinal axis of the protruding part of the ear aligning member. Te ear aligning member and the first connector may be synchronously slidable relative to the lateral end of the main body. This allows an operator to adjust the position of the ear aligning member for fitting the protruding part of the ear aligning member into an ear canal of the subject.

According to certain embodiments of the present invention, the head fixation assembly further includes a pillow member for supporting the head of the subject, the pillow member being disposed on the main body between its lateral ends. Preferably, the pillow member is adapted for adjusting a tilt angle of the head of the subject with respect to a transverse plane of the subject. In addition, the pillow member may be replaceable.

The guide can be configured such that the line representing the first reference plane is orthogonal to both the longitudinal axis of the protruding part of the ear aligning member and the base. In other words, the first reference plane passes through the first pivot point, and thus, the opening of ear canal of the subject. The first reference plane can be a Frankfort horizontal plane of the subject. The Frankfort horizontal plane may be defined as a line drawn from (the superior aspect of) the external auditory canal to the most inferior aspect of the infraorbital rim. Accordingly, in such cases, an operator can use the guide(s) to assist the positioning of the head of the subject, by fitting the protruding part(s) of the ear aligning member(s) into the ear canal(s) of the subject and then aligning an end point of the line of the guide(s) with the most inferior aspect of the infraorbital rim(s) of the subject, to adjust the subject's head (for example, adjusting the tilt angle of the head by using the pillow member) to a position where a Frankfort horizontal plane of the subject is orthogonal to the base.

According to certain preferred embodiments, the first stand includes a first arm extending upward from the base to an upper end; a second arm connected to the upper end of the first arm at a right angle and extending along a direction parallel to the medial line of the head fixation assembly to an inferior end; a third arm connected to the inferior end of the second arm at a right angle and extending along a direction perpendicular to the medial line of the head fixation assembly to an medial end; and a fourth arm connected to the medial end of the third arm at a right angle and extending downward to a lower end. Preferably, the length of each arm is adjustable. In one embodiment, the holder is pivotally connected to the lower end of the fourth arm. The holder can be customized or adjustable for different assessment/intervention purposes. For example, some holders may be suitable for "transverse ultrasound scanning", and other holders may be suitable for "sagittal ultrasound scanning". Alternatively, for example, the holder may be rotatable with respect to the fourth arm for the switch between a "transverse ultrasound scanning" mode and a "sagittal ultrasound scanning" mode.

Considering individual differences, the ear aligning member can be adjustable in the sense that its distance to the ear of the subject can be adjusted.

According to certain embodiments of the present invention, the positioning apparatus further comprises a second light transmitter. The second light transmitter is able to project a second light beam which defines a third reference plane. In some preferred embodiments, the third reference plane represents a medial plane of the subject. In one embodiment of the present invention, the second light transmitter is configured on the fourth arm of the first stand on a side facing the head fixation assembly.

In certain embodiments of the present invention, the positioning apparatus further comprises a second stand, a second connector, and a second light transmitter. The second stand is secured to the base and extends upward to an upper end. The second connector has a first end and an opposite second end. The first end of the second connector is pivotally connected to the upper end of the second stand at a second pivot point, such that the second connector is rotatable with respect to the second pivot point. The second light transmitter is able to project a second light beam and is configured at the second end of the second connector, such that the second light beam defines a third reference plane passing through the second pivot point. In one embodiment of the present invention, the second stand is secured to the base at a securing point where a medial line of the head fixation assembly passes through. In such embodiment, the third reference plane may be a medial plane of the subject.

However, instead of securing to the base, the second stand can be constructed as a separate module from the positioning apparatus, allowing an operator to set up the second stand and the second light transmitter thereon at a desired location where the second light beam is properly projected onto the subject to delineate a third reference plane.

Furthermore, alternatively, the second light transmitter may be configured on the fourth arm (e.g., at a side facing the head fixation assembly) of the above-mentioned first stand. Preferably, the second light transmitter is adjustable with respect to the fourth arm, so that an operator can adjust the third reference plane defined by the second light beam.

The first connector and/or the second connector can in some cases be retractable in length, so that an operator can adjust the first light transmitter and/or the second light transmitter to a desired location.

Preferably, the first light transmitter is a laser transmitter which projects a linear laser beam, and the second light transmitter is also a laser transmitter which projects a linear laser beam.

The first light beam defining the second reference plane and the second light beam defining the third reference plane may have a cross-point on the subject, representing a desired point where the assessment or intervention is to be performed. For example, an operator can move the medical device to direct to said cross-point using the adjustable first stand of the holder assembly, to perform a specific assessment or intervention.

Accordingly, the first stand preferably provides some degree of freedom of movement of the medical device, such that an operator can move the medical device to an appropriate position for the assessment or intervention, through a computer controlled system or manually.

In some embodiments of the present invention, the holder assembly further includes a motor for rotating the holder with respect to the first stand. Such embodiments are especially useful in 3D ultrasound imaging which requires a range of scanning. The movement/rotation patterns of the holder may be controlled by a computer program.

According to certain embodiments of the present invention, the holder comprises a frame part and a holding part, the frame part being pivotally connected to the first stand, the holding part being pivotally connected to the frame part and adapted for holding the medical device. In one embodiment, the holder further comprises a motor for rotating the holding part with respect to the frame part, such that the tip of the medical device can be moved toward a superior direction and/or an inferior direction of the subject. Further, the holding part may be coaxially rotatable with respect to the frame part for the switch between a "transverse ultrasound scanning" mode and a "sagittal ultrasound scanning" mode, in the case of ultrasound scanning, but not limited thereto. In another embodiment, the holding part includes a cushioning element for cushioning the movement of the medical device relative to the holding part.

In certain preferred embodiments of the present invention, the holder assembly further includes a cushioning element for cushioning the movement of the medical device relative to the holder. This makes it less uncomfortable when the medical device is required to be held close against and/or move around an assessment area on the subject.

According to one preferred embodiment of the present invention, the positioning apparatus is for use in the assessment of obstructive sleep apnea of the subject by ultrasound imaging. In such embodiment, the second reference plane may be a hyoid-external auditory meatus plane of the subject, and the third reference plane may be a medial plane of the subject.

The positioning apparatus may also be used in at least the following assessments or interventions: ultrasound-guided interventions (e.g., puncture, injection, biopsy, and nerve block), and ultrasound imaging of peripheral arteriovenous of neck, carotid artery, cervical lymph node, thyroid, parathyroid or parotid.

Certain preferred embodiments of the present invention are described below.

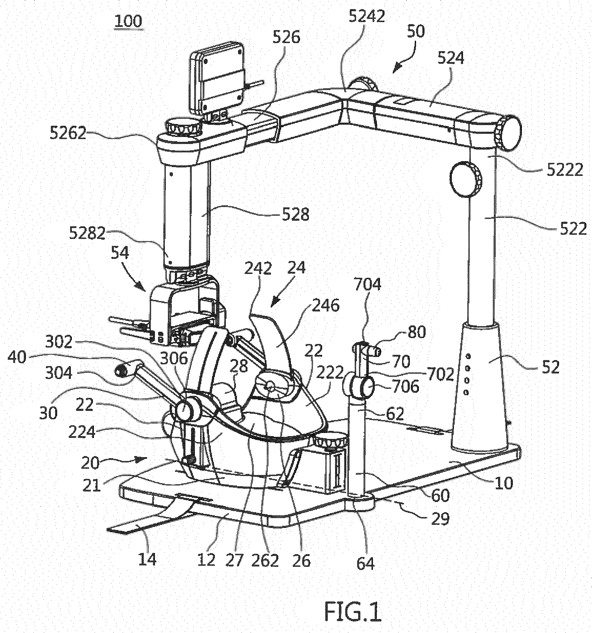

With reference to Figs. 1-3, provided is a positioning apparatus **100** for head and neck assessment or intervention of a subject. The positioning apparatus **100** comprises a base **10**, a head fixation assembly **20**, a first connector **30**, a first light transmitter **40**, a holder assembly 50**,** a second stand **60**, a second connector **70**, and a second light transmitter **80**. The head fixation assembly **20** includes a main body **21**, a guide **24** representing a first reference plane **92** of the subject, and an ear aligning member **26**. The main body **21** is attached to the base **10** and has two upwardly extending lateral ends **22**. The guide **24** extends upward and slightly inward from each of the lateral ends **22**. The ear aligning member **26** is configured on an inner side **222** of each of the lateral ends **22** and has a protruding part **262** for fitting into the ear canal of the subject. The head fixation assembly **20** may further include a pillow member **28** for supporting the head of the subject, the pillow member **28** being disposed on the main body **21** between its lateral ends **22**. Preferably, the pillow member is **28** adapted for adjusting a tilt angle of the head of the subject with respect to a transverse plane of the subject.

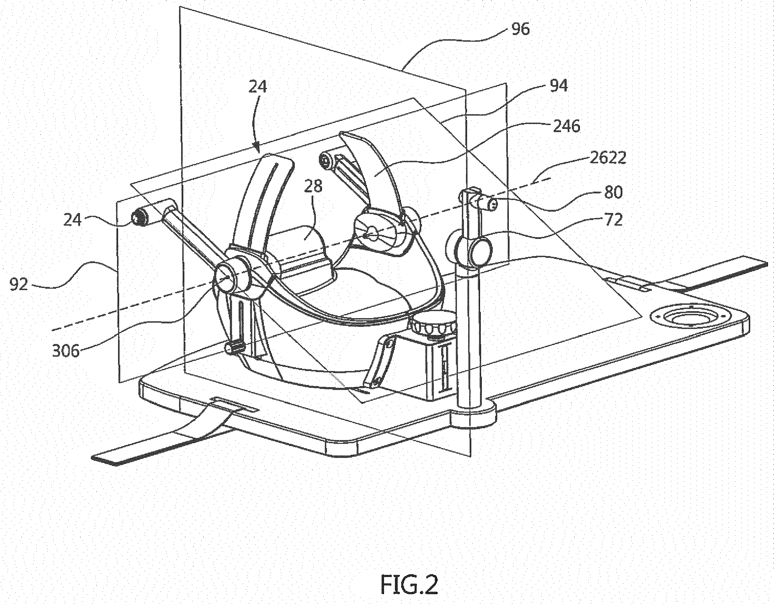

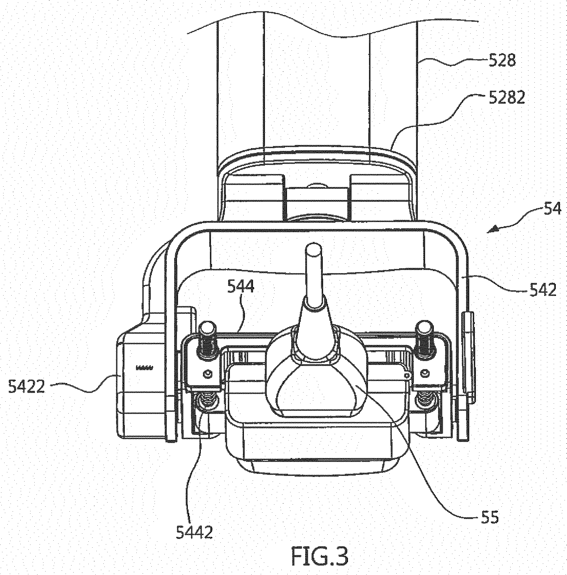

The first connector **30** has a first end **302** and an opposite second end **304**. The first end **302** is pivotally connected to the lateral end **22** of the main body **21** on an outer side **224** at a first pivot point **306** lying on a longitudinal axis **2622** (see Fig. 2) of the protruding part **262** of the ear aligning member **26**. The first light transmitter **40** is able to project a first light beam, and is configured at the second end **304** of the first connector **30**, such that the first light beam defines a second reference plane **94** (see Fig. 2) passing through the first pivot point **306**. The holder assembly **50** includes an adjustable first stand **52**, and a holder **54** for holding a medical device **55** for the assessment or intervention (see Fig. 1 and Fig. 3). The first stand **52** is secured to the base **10**, and the holder **54** is pivotally connected to the first stand **52**. The second stand **60** is secured to the base **10** and extends upward to an upper end **62**. The second connector **70** has a first end **702** and an opposite second end **704**. The first end **702** of the second connector **70** is pivotally connected to the upper end **62** of the second stand **60** at a second pivot point **706**. The second light transmitter **80** is able to project a second light beam and is configured at the second end **704** of the second connector **70**, such that the second light beam defines a third reference plane **96** passing through the second pivot point **706**. In this embodiment, the second stand **60** is secured to the base **10** at a securing point **64** where a medial line **29** of the head fixation assembly **20** passes through. As such, the third reference plane **96** may be a medial plane of the subject if the second connector **70** is in an upright position. In addition, the first stand **52** includes a first arm **522** extending upward from the base **10** to an upper end **5222**; a second arm **524** connected to the upper end **5222** of the first arm **522** at a right angle and extending along a direction parallel to the medial line **29** of the head fixation assembly **20** to an inferior end **5242**; a third arm **526** connected to the inferior end **5242** of the second arm **524** at a right angle and extending along a direction perpendicular to the medial line **29** of the head fixation assembly **20** to an medial end **5262**; and a fourth arm **528** connected to the medial end **5262** of the third arm **526** at a right angle and extending downward to a lower end **5282**. The holder **54** includes a frame part **542** and a holding part **544**, the frame part **542** being pivotally connected to the lower end **5282** of the fourth arm **528** of the first stand **50**, the holding part **544** being pivotally connected to the frame part **542** and adapted for holding the medical device **55** (see Fig. 3). The holder **54** further includes a motor **5422** for rotating the holding part **544** with respect to the frame part **542**. The holding part **544** includes a cushioning element **5442** for cushioning the movement of the medical device **55** relative to the holding part **544**.

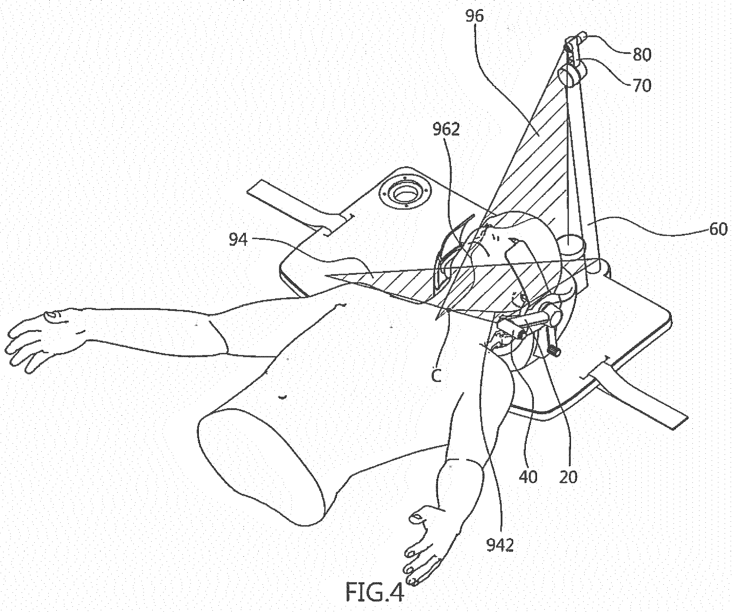

Fig. 4 is a perspective drawing illustrating the use of a positioning apparatus according to the present invention in the assessment of obstructive sleep apnea of a subject by ultrasound imaging. Referring to Fig. 4 together with Figs. 1-2, after the subject lies down with his/her head placed in the head fixation assembly **20** on the pillow member **28**, and the positions of both ear aligning members **26** is adjusted to align a respective opening of ear canal of the subject, the tilt angle of the subject's head may be first adjusted with the aid of the guide **24** through the pillow member **28** such that the subject's Frankfort horizontal plane (represented by the first reference plane **92**) is parallel to the normal direction the base (and thus perpendicular to the surface of the examination bed (not shown)). Subsequently, the operator may identify the location of the subject's hyoid by hand and adjust the first connector **30** accordingly such that the second reference plane **94** defined by the first light beam from the first light transmitter **40** represents the hyoid-external auditory meatus plane of the subject and that the first light beam delineates a first line **942** (passing through the identified location of the hyoid) on the subject's surface. Further, the second connector **70** may be adjusted to an upright position such that the third reference plane **96** defined by the second light beam from the second light transmitter **80** represents the medial plane of the subject and that the second light beam delineates a second line **962** on the subject's surface. The first and second lines **942** and **962** would cross at a point **C** on the subject's surface. Then, the operator may adjust the adjustable first stand **52** to direct the tip of an ultrasound probe to the point **C** for scanning the retroglossal region and the retropalatal region of the subject. In another example, for thyroid examination, the operator may identify the location of the subject's thyroid cartilage instead, and adjust the first connector **30** accordingly such that the second reference plane **94** defined by the first light beam from the first light transmitter **40** represents a thyroid cartilage-external auditory meatus plane of the subject and that the first light beam delineates a first line **942** (passing through the identified location of the thyroid cartilage) on the subject's surface. As such, the point **C** where the first and second lines **942** and **962** cross represents a central point of the subject's thyroid cartilage toward which the operator may adjust the adjustable first stand **52** to direct the tip of an ultrasound probe for thyroid examination.

In certain preferred embodiments of the present invention, the holder assembly further includes a cushioning element for cushioning the movement of the medical device relative to the holder. This makes it less uncomfortable when the medical device is required to be held close against and/or move around an assessment area on the subject.

## Claims

1. A positioning apparatus (100) for head and neck assessment or intervention of a subject, comprising:
a base (10);
a head fixation assembly (20) including:
a main body (21) attached to the base (10), the main body (21) having two upwardly extending lateral ends (22);
a guide (24) representing a first reference plane (92) of the subject, the guide (24) extending upward and slightly inward from each of the lateral ends (22); and
a holder assembly (50) including:
an adjustable first stand (52) secured to the base (10); and
a holder (54) for holding a medical device (55) for the assessment or intervention, pivotally connected to the first stand (52),
**characterized in that** the positioning apparatus (100) further comprises
an ear aligning member (26) for aligning with the opening of ear canal of the subject, configured on an inner side (222) of each of the lateral ends (22);
a first connector (30) having a first end (302) and an opposite second end (304), the first end (302) being pivotally connected to one of the lateral ends (22) of the main body (21) on an outer side (224) at a first pivot point (306); and
a first light transmitter (40) for projecting a first light beam, configured at the second end (304) of the first connector (30), such that the first light beam defines a second reference plane (94) passing through the first pivot point (306).

2. The positioning apparatus (100) of claim 1, further comprising:
a second stand (60) secured to the base (10) and extending upward to an upper end (62);
a second connector (70) having a first end (702) and an opposite second end (704), the first end (702) being pivotally connected to the upper end (62) of the second stand (60) at a second pivot point (706); and
a second light transmitter (80) for projecting a second light beam, configured at the second end (704) of the second connector (70), such that the second light beam defines a third reference plane (96) passing through the second pivot point (706).

3. The positioning apparatus (100) of claim 1, wherein the guide (24) includes a line representing the first reference plane (92), or wherein the guide includes a transparent substrate extending upward and slightly inward from each of the lateral ends (22).

4. The positioning apparatus (100) of claim 3, wherein the transparent substrate is marked with a line representing the first reference plane (92), or projected with a light beam which serves as a line representing the first reference plane.

5. The positioning apparatus (100) of claim 1, wherein the first light transmitter (40) is a laser transmitter projecting a linear laser beam, and/or wherein the second light transmitter (80) is a laser transmitter projecting a linear laser beam.

6. The positioning apparatus (100) of claim 1, wherein the main body (21) of the head fixation assembly (20) is slidably attached to the base (10).

7. The positioning apparatus (100) of claim 1, wherein the head fixation assembly (20) further includes a pillow member (28) for supporting the head of the subject, disposed on the main body (21) between its lateral ends (22).

8. The positioning apparatus (100) of claim 7, wherein the pillow member (28) is adapted for adjusting a tilt angle of the head of the subject with respect to a transverse plane of the subject.

9. The positioning apparatus (100) of claim 1, wherein the first stand (52) includes:
a first arm (522) extending upward from the base (10) to an upper end (5222);
a second arm (524) connected to the upper end (5222) of the first arm (522) at a right angle and extending along a direction parallel to the medial line (29) of the head fixation assembly (20) to an inferior end (5242);
a third arm (526) connected to the inferior end (5242) of the second arm (524) at a right angle and extending along a direction perpendicular to the medial line (29) of the head fixation assembly (20) to an medial end (5262); and
a fourth arm (528) connected to the medial end (5262) of the third arm (526) at a right angle and extending downward to a lower end (5282),
wherein the holder (54) is pivotally connected to the lower end (5282) of the fourth arm (528).

10. The positioning apparatus of claim 1, wherein the holder (54) assembly further includes a motor (5422) for rotating the holder (54) with respect to the first stand (52), and/or wherein the holder assembly (50) further includes a cushioning element (5442) for cushioning the movement of the medical device (55) relative to the holder (54).

11. The positioning apparatus of claim 1, wherein the holder (54) includes a frame part (542), a holding part (544) and a motor (5422) for rotating the holding part (544) with respect to the frame part (542), the frame part (542) being pivotally connected to the first stand (52), the holding part (544) being pivotally connected to the frame part (542) and adapted for holding the medical device (55).

12. The positioning apparatus (100) of claim 11, wherein the holder (54) further includes a motor (5422) for rotating the holding part (544) with respect to the frame part (542), and/or wherein the holding part (544) includes a cushioning element (5442) for cushioning the movement of the medical device (55) relative to the holding part (544).

13. The positioning apparatus (100) of claim 1 for use in the assessment of obstructive sleep apnea by ultrasound imaging.

14. The positioning apparatus (100) of claim 9, wherein the length of each arm (522, 524, 526, 528) is adjustable, and/or wherein the head fixation assembly (20) further includes a pillow member (28) for supporting the head of the subject, disposed on the main body (21) between the lateral ends (22), the pillow member (28) being adapted for adjusting a tilt angle of the head of the subject with respect to a transverse plane of the subject.

15. The positioning apparatus (100) of claim 14, further comprising a computer module adapted to detect and memorize the length of each arm (522, 524, 526, 528), and/or the position of the pillow member (28) corresponding to a tilt angle of the head of the subject with respect to a transverse plane of the subject.

## Patentansprüche

1. Positioniervorrichtung (100) zur Untersuchung oder zum Eingriff in den Kopf- und Nackenbereich eines Individuums, welche umfasst:
eine Basis (10);
einen Kopf-Fixierungs-Aufbau (20), der einschließt:
einen an der Basis (10) angefügten Hauptkörper (21), worin der Hauptkörper (21) zwei sich aufwärts erstreckende Seitenenden (22) aufweist;
eine Führung (24), die eine erste Bezugsebene (92) des Patienten repräsentiert, worin sich die Führung (24) aufwärts und von jedem der Seitenenden (22) leicht einwärts erstreckt; und
einen Halterungs-Aufbau (50), der einschließt:
einen an der Basis (10) befestigten regelbaren ersten Ständer (52); und
eine drehbar mit dem ersten Ständer (52) verbundene Halterung (54) zum Halten eines medizinischen Gerätes (55) für die Untersuchung oder den Eingriff,
**dadurch gekennzeichnet, dass** die Positioniervorrichtung (100) weiter umfasst:
ein Ohr-Ausrichtungs-Element (26) zur Ausrichtung mit der Öffnung des Ohrkanals des Individuums, das an einer Innenseite (222) jeder der Seitenenden (22) angeordnet ist;
ein erstes Verbindungsstück (30), das ein erstes Ende (302) und ein abgewandtes zweites Ende (304) aufweist, worin das erste Ende (302) mit einem der Seitenenden (22) des Hauptkörpers (21) an einer Außenseite (224) an einem ersten Drehpunkt (306) drehbar verbunden ist; und
einen an dem zweiten Ende (304) des ersten Verbindungsstückes (30) zum Projizieren eines ersten Lichtstrahls derart ausgestalteten ersten Lichtsender (40), dass der erste Lichtstrahl eine zweite Bezugsebene (94) definiert, die durch den ersten Drehpunkt (306) geht.

2. Positioniervorrichtung (100) nach Anspruch 1, welche weiter umfasst:
einen an der Basis (10) befestigten und sich aufwärts zu einem oberen Ende (62) erstreckenden zweiten Ständer (60);
ein zweites Verbindungsstück (70), das ein erstes Ende (702) und ein abgewandtes zweites Ende (704) aufweist, worin das erste Ende (702) mit dem oberen Ende (62) des zweites Ständers (60) an einem zweiten Drehpunkt (706) drehbar verbunden ist; und
einen zweiten Lichtsender (80) zum Projizieren eines zweiten Lichtstrahls, der an dem zweiten Ende (704) des zweiten Verbindungsstücks (70) so ausgestaltet ist, dass der zweite Lichtstrahl eine dritte Bezugsebene (96) definiert, die durch den zweiten Drehpunkt (706) geht.

3. Positioniervorrichtung (100) nach Anspruch 1, worin die Führung (24) eine Linie umfasst, die die erste Bezugsebene (92) repräsentiert oder worin die Führung ein transparentes Substrat umfasst, das sich von jedem der Seitenenden (22) aufwärts und leicht einwärts erstreckt.

4. Positioniervorrichtung (100) nach Anspruch 3, worin das transparente Substrat mit einer Linie gekennzeichnet ist, die die erste Bezugsebene (92) repräsentiert oder mit einem Lichtstrahl projiziert wird, der als eine die erste Bezugsebene repräsentierende Linie dient.

5. Positioniervorrichtung (100) nach Anspruch 1, worin der erste Lichtsender (40) ein Laser-Überträger ist, der einen linearen Laserstrahl projiziert und/oder worin der zweite Lichtsender (80) ein Laser-Überträger ist, der einen linearen Laserstrahl projiziert.

6. Positioniervorrichtung (100) nach Anspruch 1, worin der Hauptkörper (21) des Kopf-Fixierungs-Aufbaus (20) gleitend an der Basis (10) angebracht ist.

7. Positioniervorrichtung (100) nach Anspruch 1, worin der Kopf-Fixierungs-Aufbau (20) zur Unterstützung des Kopfes des Patienten weiter ein Polster-Element (28) umfasst, das auf dem Hauptkörper (21) zwischen seinen Seitenenden (22) angeordnet ist.

8. Positioniervorrichtung (100) nach Anspruch 7, worin das Polster-Element (28) zum Ausrichten eines Kippwinkels des Kopfes des Patienten im Bezug zu einer Querebene des Patienten angepasst ist.

9. Positioniervorrichtung (100) nach Anspruch 1, worin der erste Ständer (52) umfasst:
einen ersten Arm (522), der sich von der Basis (10) zu einem oberen Ende (5222) aufwärts erstreckt;
einen zweiten Arm (524), der mit dem oberen Ende (5222) des ersten Armes (522) in einem rechten Winkel verbunden ist und sich entlang einer zu der Mittellinie (29) des Kopf-Fixierungs-Aufbaus (20) parallelen Richtung zu einem hinteren Ende (5242) erstreckt;
einen dritten Arm (526), der mit dem hinteren Ende (5242) des zweiten Armes (524) in einem rechten Winkel verbunden ist und sich entlang einer zu der Mittellinie (29) des Kopf-Fixierungs-Aufbaus (20) senkrechten Richtung zu einem mittleren Ende (5262) erstreckt; und
einen vierten Arm (528), der mit dem mittleren Ende (5262) des dritten Armes (526) in einem rechten Winkel verbunden ist und sich zu einem unteren Ende (5282) abwärts erstreckt,
worin die Halterung (54) mit dem unteren Ende (5282) des vierten Armes (528) drehbar verbunden ist.

10. Positioniervorrichtung nach Anspruch 1, worin der Halterungs (54) -Aufbau weiter einen Motor (5422) zum Drehen der Halterung (54) in Bezug zu dem ersten Ständer (52) umfasst, und/oder worin der Halterungs-Aufbau (50) weiter ein Dämpfungs-Element (5442) zum Dämpfen der Bewegung des medizinischen Gerätes (55) relativ zu der Halterung (54) umfasst.

11. Positioniervorrichtung nach Anspruch 1, worin die Halterung (54) ein Rahmen-Teil (542), ein Halterungs-Teil (544) und einen Motor (5422) zum Drehen der Halterungsteile (544) in Bezug zu dem Rahmen-Teil (542) umfasst, worin das Rahmen-Teil (542) mit dem ersten Ständer (52) drehbar verbunden ist, worin das Halterungs-Teil (544) mit dem Rahmen-Teil (542) drehbar verbunden und angepasst ist, das medizinische Gerät (55) zu halten.

12. Positioniervorrichtung (100) nach Anspruch 11, worin die Halterung (54) weiter einen Motor (5422) zum Drehen des Halterungs-Teils (544) in Bezug zu dem Rahmen-Teil (542) umfasst, und/oder worin das Halterungs-Teil (544) ein Dämpfungs-Element (5442) zum Dämpfen der Bewegung des medizinischen Gerätes (55) relativ zu dem Halterungs-Teil (544) umfasst.

13. Positioniervorrichtung (100) nach Anspruch 1 zur Verwendung bei der Untersuchung von obstruktiver Schlaf-Apnoe durch Ultraschall-Bildgebung.

14. Positioniervorrichtung (100) nach Anspruch 9, worin die Länge jedes Armes (522, 524, 526, 528) eingestellt werden kann, und/oder worin der Kopf-Fixierungs-Aufbau (20) weiter ein Polster-Element (28) zum Unterstützen des Kopfes des Patienten umfasst, das auf dem Hauptkörper (21) zwischen den Seitenenden (22) angeordnet ist, worin das Polster-Element (28) angepasst ist, einen Kippwinkel des Kopfes des Patienten im Bezug zu einer Querebene des Patienten auszurichten.

15. Positioniervorrichtung (100) nach Anspruch 14, welche weiter ein Rechner-Modul umfasst, das zum Erfassen und Speichern der Länge jedes Armes (522, 524, 526, 528) und/oder der einem Kipp-Winkel des Kopfes des Patienten im Bezug zu einer Querebene des Patienten entsprechenden Position des Polster-Elements (28) angepasst ist.

## Revendications

1. Appareil de positionnement (100) pour l'évaluation ou l'intervention sur la tête et le cou d'un sujet, comprenant :
une base (10) ;
un ensemble de fixation de tête (20) comprenant :
un corps principal (21) attaché à la base (10), le corps principal (21) ayant deux extrémités latérales s'étendant vers le haut (22) ;
un guide (24) représentant un premier plan de référence (92) du sujet, le guide (24) s'étendant vers le haut et légèrement vers l'intérieur à partir de chacune des extrémités latérales (22) ; et
un ensemble de dispositif de maintien (50) comprenant :
un premier support réglable (52) fixé à la base (10) ; et
un dispositif de maintien (54) destiné à maintenir un dispositif médical (55) pour l'évaluation ou l'intervention, relié de manière pivotante au premier support (52), **caractérisé en ce que** l'appareil de positionnement (100) comprend en outre
un élément d'alignement d'oreille (26) destiné à être aligné avec l'ouverture du conduit auditif du sujet, configuré sur un côté interne (222) de chacune des extrémités latérales (22) ;
un premier connecteur (30) ayant une première extrémité (302) et une seconde extrémité opposée (304), la première extrémité (302) étant reliée de manière pivotante à l'une des extrémités latérales (22) du corps principal (21) sur un côté externe (224) au niveau d'un premier point de pivotement (306) ; et
un premier émetteur de lumière (40) destiné à projeter un premier faisceau de lumière, configuré au niveau de la seconde extrémité (304) du premier connecteur (30), de telle sorte que le premier faisceau lumineux définit un deuxième plan de référence (94) passant par le premier point de pivotement (306).

2. Appareil de positionnement (100) selon la revendication 1, comprenant en outre :
un deuxième support (60) fixé à la base (10) et s'étendant vers le haut jusqu'à une extrémité supérieure (62) ;
un deuxième connecteur (70) ayant une première extrémité (702) et une seconde extrémité opposée (704), la première extrémité (702) étant reliée de manière pivotante à l'extrémité supérieure (62) du deuxième support (60) au niveau d'un deuxième point de pivotement (706) ; et
un deuxième émetteur de lumière (80) destiné à projeter un deuxième faisceau de lumière, configuré au niveau de la seconde extrémité (704) du deuxième connecteur (70), de telle sorte que le deuxième faisceau lumineux définit un troisième plan de référence (96) passant par le deuxième point de pivotement (706).

3. Appareil de positionnement (100) selon la revendication 1, dans lequel le guide (24) comprend une ligne représentant le premier plan de référence (92), ou dans lequel le guide comprend un substrat transparent s'étendant vers le haut et légèrement vers l'intérieur à partir de chacune des extrémités latérales (22).

4. Appareil de positionnement (100) selon la revendication 3, dans lequel le substrat transparent est marqué avec une ligne représentant le premier plan de référence (92), ou projeté avec un faisceau lumineux qui sert de ligne représentant le premier plan de référence.

5. Appareil de positionnement (100) selon la revendication 1, dans lequel le premier émetteur de lumière (40) est un émetteur laser projetant un faisceau laser linéaire, et/ou dans lequel le deuxième émetteur de lumière (80) est un émetteur laser projetant un faisceau laser linéaire.

6. Appareil de positionnement (100) selon la revendication 1, dans lequel le corps principal (21) de l'ensemble de fixation de tête (20) est attaché de manière coulissante à la base (10).

7. Appareil de positionnement (100) selon la revendication 1, dans lequel l'ensemble de fixation de tête (20) comprend en outre un élément d'oreiller (28) pour soutenir la tête du sujet, disposé sur le corps principal (21) entre ses extrémités latérales (22).

8. Appareil de positionnement (100) selon la revendication 7, dans lequel l'élément d'oreiller (28) est conçu pour régler un angle d'inclinaison de la tête du sujet par rapport à un plan transversal du sujet.

9. Appareil de positionnement (100) selon la revendication 1, dans lequel le premier support (52) comprend :
un premier bras (522) s'étendant vers le haut à partir de la base (10) jusqu'à une extrémité supérieure (5222) ;
un deuxième bras (524) relié à l'extrémité supérieure (5222) du premier bras (522) selon un angle droit et s'étendant le long d'une direction parallèle à la ligne médiane (29) de l'ensemble de fixation de tête (20) jusqu'à une extrémité inférieure (5242) ;
un troisième bras (526) relié à l'extrémité inférieure (5242) du deuxième bras (524) selon un angle droit et s'étendant le long d'une direction perpendiculaire à la ligne médiane (29) de l'ensemble de fixation de tête (20) jusqu'à une extrémité médiale (5262) ; et
un quatrième bras (528) relié à l'extrémité médiale (5262) du troisième bras (526) selon un angle droit et s'étendant vers le bas jusqu'à une extrémité inférieure (5282),
dans lequel le dispositif de maintien (54) est relié de manière pivotante à l'extrémité inférieure (5282) du quatrième bras (528).

10. Appareil de positionnement selon la revendication 1, dans lequel l'ensemble de dispositif de maintien (54) comprend en outre un moteur (5422) pour faire tourner le dispositif de maintien (54) par rapport au premier support (52), et/ou dans lequel l'ensemble de dispositif de maintien (50) comprend en outre un élément d'amortissement (5442) pour amortir le mouvement du dispositif médical (55) par rapport au dispositif de maintien (54).

11. Appareil de positionnement selon la revendication 1, dans lequel le dispositif de maintien (54) comprend une partie de cadre (542), une partie de maintien (544) et un moteur (5422) pour faire tourner la partie de maintien (544) par rapport à la partie de cadre (542), la partie de cadre (542) étant reliée de manière pivotante au premier support (52), la partie de maintien (544) étant reliée de manière pivotante à la partie de cadre (542) et conçue pour maintenir le dispositif médical (55).

12. Appareil de positionnement (100) selon la revendication 11, dans lequel le dispositif de maintien (54) comprend en outre un moteur (5422) pour faire tourner la partie de maintien (544) par rapport à la partie de cadre (542), et/ou dans lequel la partie de maintien (544) comprenant un élément d'amortissement (5442) pour amortir le mouvement du dispositif médical (55) par rapport à la partie de maintien (544),

13. Appareil de positionnement (100) selon la revendication 1 destiné à être utilisé dans l'évaluation de l'apnée obstructive du sommeil par imagerie ultrasonore.

14. Appareil de positionnement (100) selon la revendication 9, dans lequel la longueur de chaque bras (522, 524, 526, 528) est réglable, et/ou dans lequel l'ensemble de fixation de tête (20) comprend en outre un élément d'oreiller (28) pour soutenir la tête du sujet, disposé sur le corps principal (21) entre les extrémités latérales (22), l'élément d'oreiller (28) étant conçu pour régler un angle d'inclinaison de la tête du sujet par rapport à un plan transversal du sujet.

15. Appareil de positionnement (100) selon la revendication 14, comprenant en outre un module informatique conçu pour détecter et mémoriser la longueur de chaque bras (522, 524, 526, 528) et/ou la position de l'élément d'oreiller (28) correspondant à un angle d'inclinaison de la tête du sujet par rapport à un plan transversal du sujet.
